# EUROPEAN PATENT APPLICATION

(11) **EP 3 640 323 A2**
(43) Date of publication of application: **22.04.2020**
(21) Application number: 18817362.9
(22) Date of filing: 12.06.2018
(51) Int. Cl.: C12N 1/21, C12N 15/11

(54) **PRODUCTION OF ROUGH-TYPE SALMONELLA ENTERITIDIS AND THE GENETIC MODIFICATIONS THEREOF FOR USE AS AN AVIAN VACCINE**

(30) Priority: 12.06.2017 PE 0009922017
(71) Applicant: Farmacologicos Veterinarios Sac, C.P. 11702 (PE)
(72) Inventor: LATASA OSTA, Cristina, 31192 Multiva Navarra (ES)
(74) Representative: Ungria López, Javier
(86) International application number: PCT/PE2018/000014
(87) International publication number: WO 2018/231078

(57) **Abstract**

The present invention relates to a strain of *Salmonella enteritidis* 3934vac from which the waaL gene has been deleted in order to obtain a rough phenotype (3934vac DwaaL), as well as to the method for obtaining same and to the oligos used, with a view to reducing toxicity and maintaining immunogenicity for the use of same as a vaccine. The present invention also relates to a strain of *Salmonella enteritidis* 3934vac DwaaL, i.e. of the rough-type, which has been modified to express the fiber gene of type-I avian adenovirus, as well as to the method for obtaining a strain of *Salmonella enteritidis* 3034 vac DwaaL which expresses a fiber gene of Ava-I. The invention further comprises the development of a novel avian vaccine against the AvA-I virus, which is live, recombinant, effective and harmless and which was developed using a process involving inserting and integrating Ava-I fiber genes into the chromosome of a non-pathogenic attenuated strain of *Salmonella enteritidis* bacteria.

## Description

### SECTOR OF THE ART

The present invention is comprised in the animal health sector. More specifically, the invention relates to the development of a rough phenotype strain of attenuated *Salmonella* enterica serovar Enteritidis (Salmonella Enteritidis or SE), obtained by deleting a gene involved in the synthesis of lipopolysaccharide. In this context, use of this strain as a vector vaccine against Ava-I virus is further included, using a process involving inserting and integrating the genes encoding the Ava-I fiber antigen into its chromosome. The SE vector that is used has been genetically modified to work as an expression vehicle of the immunodominant fiber genes of the AvA-I virus so as to stimulate an effective and long-lasting immune response against IBH (viral inclusion body hepatitis) in poultry. The generated strain is completely safe because it is missing the twelve genes encoding the proteins of the signaling pathway of the second messenger cyclic di-GMP, the sigma factor RpoS and the protein WaaL. Therefore, this strain is proposed as a novel vaccine against the Ava-I virus, which is live, recombinant, effective and harmless, and which is suitable for vaccinating poultry populations.

All the components of this vaccine have specifically been designed and developed bearing in mind the three priority factors of efficacy, safety and costs.

### BACKGROUND OF THE INVENTION

A 30 % growth in the poultry sector is expected for the next 8 to 10 years (APA, 2008), accompanied by successful health programs which in turn depend on effective and safe vaccines. IBH/HPS disease causes mortality in up to 50 % of all affected poultry and is a latent problem in Peru because outbreaks still exist despite vaccination, which is an impossible problem to solve given that the technology used to produce the vaccines is the same as it was 20 years ago.

Recently, genetic manipulation techniques combined with bacterial genome availability, greater knowledge about the mechanisms of pathogenesis and the immune response have allowed new approaches for the development of attenuated bacterial strains as heterologous gene vectors. Thus, enteropathogenic bacteria such as *Listeria monocytogenes,* Salmonella spp, Shigella spp, *Vibrio cholerae, Yersinia enterocolitica or Bordetella pertussis* have been used as vectors effective as vaccines. Among these bacteria, Salmonella spp has yielded the most promising results. Thus, vector vaccines based on attenuated strains of *Salmonella typhimurium* have been used for generating a protective immune response against viral, bacterial and protozoan pathogens and incorporating anti-cancer treatments into vehicles. In the specific case of poultry, this type of vaccine has been evaluated as a gene vector of the infectious bronchitis virus, avian influenza virus, avian reovirus and *Clostridium perfringens.* All these papers agree that these vaccines were capable of provoking a long-lasting protective immune response, in addition to being safe as they prevent the risk of causing the disease. Considering the aforementioned aspects, there is substantial support for the success that the development of a live recombinant vaccine against AvA-I using a strain of attenuated SE as a vector would offer in the poultry industry. These advantages can be summarised as: (1) severe attenuation as a result of several targeted mutagenesis processes through a mechanism of allelic exchange which prevents reversion, they do not introduce exogenous DNA and convert the resulting strain into a totally safe vector vaccine; (2) the amount of expression of the AvA-I antigen can be regulated by means of genetic manipulation; (3) it can be administered by oral inoculation; (4) it strongly stimulates the innate and the adaptive immune system; and (5) it would in turn afford protection against SE. Although the development of vaccines using bacterial vectors has been and is currently the object of a number of studies, until now there have been no licensed SE-based commercial vaccines. Therefore, the development of this vaccine would set a good precedent in this field of research and development and would not have any competition on the national and international market.

The strain used in the present invention was isolated and described by (Solano et al., 2002). This study reports a new selection method based on the fluorescence of colonies on calcofluor agar plates to identify mutants of *Salmonella enteritidis* that are defective in biofilm development. These results not only confirmed the requirement of genes already described for the modulation of multicellular behaviour in *Salmonella typhimurium* and other species, but also revealed new aspects of the biofilm formation process, such as new genetic elements, named as bcsABZC and bcsEFG operons, required for the synthesis of an exopolysaccharide that can be hydrolysed by cellulase. Non-polar mutations of BcsC and bcsE genes and complementation experiments demonstrated that both operons are essential for cellulose biosynthesis in LB and ATM media, in both *S. enteritidis and S typhimurium.* This study also showed that the biofilm produced by *S. enteritidis* is made of different constituents, suggesting that the biofilm composition and regulation depends on environmental conditions. Furthermore, the results suggest that cellulose is not involved in the virulence of *S. enteritidis,* although the mutants deficient in the production of this polysaccharide were more sensitive to chlorine treatments, thus suggesting that cellulose production and biofilm formation would be an important factor for the survival of S. enteritidis on surfaces. In a later study, Solano *et al.* (2009) carried out an ambitious genetic approach in which the 12 genes encoding proteins with diguanylate cyclase (DGC, proteins with GGDEF domain) enzyme activity were deleted from the same strain described earlier. These enzymes synthesise second messenger C-di-GMP, a molecule whose involvement in key bacterial biological processes such as biofilm formation or virulence has been widely studied in a number of species. In the specific case of this strain of SE, total depletion of c-di-GMP is correlated with the loss of the capacity to form biofilms, a slight attenuation in a murine model and a higher sensitivity to extreme environmental conditions.

As a result of the genetic approach described above, patent document ES 2324084B1 describes the invention of a method which allows producing multiple modifications in the Gram-negative bacteria chromosome and strains of Salmonella deficient in C-di-GMP synthesis obtained by same. Said invention also relates to two mutant strains of Salmonella enterica obtained by means of said method in which the twelve genes encoding proteins with GGDEF domain have been deleted (mutant ΔXII::Km and mutant ΔXII::Clo), and to the use thereof as expression vectors, immunotherapeutic agents and in metabolic studies.

Garcia Ona (2011), in the dissertation entitled "Análisis de una cepa avirulenta de *Salmonella enteritidis* para su uso como vacuna viva atenuada" (Analysis of an avirulent strain of *Salmonella Enteritidis* for use as a live attenuated vaccine), studied the potential of several derived strains included in document ES 2324084B1 for the development of a vaccine capable of inducing an immune response, reducing the colonisation of organs and releasing Salmonella in feces on large-scale pig livestock farms. This paper concluded that the candidate strain of Salmonella for the development of a vaccine would be the strain known as S. Enteritidis 3934ΔXII*, in which the 12 genes encoding proteins with GGDEF domain have been deleted and which carries an additional deletion in a chromosomal region including transcriptional regulator RpoS. This strain is avirulent, presents a very low capacity to survive in the environment, and since it does not contain exogenous DNA, it would not be classified as a GMO (genetically modified organism). Oral immunisation tests using a murine model of infection revealed that immunisation with a single dose of 10⁷ cfu (colony-forming units) of this strain is capable of inducing a protective immune response against *Salmonella* infection, indicating that said strain can be a suitable candidate for use as a live attenuated vaccine.

Latasa et. al, 2016 performed the following study with a view to constructing a novel live attenuated vaccine of *Salmonella:* first, the impact of the absence of cyclic di-GMP (c-di-GMP) on the virulence of *Salmonella* was analysed. As mentioned earlier, C-di-GMP is an intracellular second messenger controlling a wide range of bacterial processes, including biofilm formation and the synthesis of virulence factors, in addition to modulating the innate immune response of the host through STING receptors. The results described in this article showed that a multiple mutant of Salmonella, from the genome of which the twelve genes encoding diguanylate cyclase proteins have been eliminated, and which, accordingly, cannot synthesise c-di-GMP, presents moderate attenuation in a murine model of systemic infection. A controlled additional mutation (unlike strain S. Enteritidis 3934ΔXII* in which the loss of this sigma factor had been the result of a natural counterselection) of the rpoS gene resulted in a synergistic attenuating effect, which led to a highly attenuated strain referred to as ΔXIII. This strain is immunogenic enough to protect mice against an oral lethal attack of a virulent strain of S. typhimurium. The immunogenicity of ΔXIII depends both on the activation of the humoral response and the cellular response and is characterised by the production of opsonising antibodies and the induction of significant levels of IFN-γ, TNF-A, IL-2, IL-17 and IL-10. ΔXIII is capable of forming biofilms and does not survive under desiccation conditions, which indicates that it could be readily eliminated from the environment. Furthermore, ΔXIII has DIVA (Differentiating Infected from Vaccinated Animals) properties, which allow differentiating infected animals from vaccinated animals. To aid with the terminology, strain DXII is also known as 3934vac.

Additionally, the document by Kong et. al, 2011 describes the effect of eliminating the O antigen and sugars from the core on the virulence and immunogenicity of *Salmonella enterica* serotype typhimurium. Non-polar deletion mutations in the waaG (rfaG), waaL (rfal), rfaH, waaJ (rfaJ), wbaP (rfbP), waaL (rfaL), or wzy (rfc) genes in a strain of wild-type S. typhimurium were performed in this study. After confirming the structure of the LPS, several *in vitro* and *in vivo* properties of each mutant were analysed, and in the end all the mutants showed significant attenuation and a lower invasion capacity compared with wild-type phenotype parent strains when they were administered to BALB/c mice by oral route. Furthermore, the strains that carried a mutation in waaG and waaL were deficient in colonisation of Peyer's patches and of the liver, this deficiency being partially compensated for after the intranasal administration of the mutant DwaaL. In the context of an attenuated strain of a vaccine supplying the pneumococcal PspA antigen, all the tested mutations translated into lower immune responses against PspA and Salmonella antigens. These results indicate that the irreversible truncation of the external core is not a viable option for the development of a live oral Salmonella vaccine, whereas a mutant wzy, which retains an O antigen unit, would be suitable for stimulating optimal protective immunity to homologous or heterologous antigens by oral, intranasal or intraperitoneal route.

### GENERAL DESCRIPTION OF THE INVENTION

The present invention relates to a strain of attenuated *Salmonella* Enteritidis which has been modified by genetic engineering techniques to delete the *waaL* gene and therefore has a rough phenotype. Another aspect of the present invention relates to said mutant strain of *Salmonella* Enteritidis in the *waaL* gene which furthermore carries the genes encoding the Ava-I fiber antigen in its chromosome. This strain confers protection against Ava-I, in addition to having a rough phenotype and being avirulent. Additionally, the invention comprises the method for obtaining a strain of attenuated *Salmonella* Enteritidis with rough phenotype and carrying out the integration of the genes encoding the AVA-I fiber into its chromosome, including the plasmids and methods used for obtaining said strain.

With a view to reducing toxicity and maintaining immunogenicity of the strains based on the 3934vac genetic background (SE DXIII), an aspect of the present invention relates to the deletion of the *waaL* gene. The mutation of this gene translates into a rough phenotype due to the fact that the lipopolysaccharide is made up of only a core and lipid A, having lost the capacity to synthesise the O antigen. This mutation has the additional advantage of allowing the differentiation of animals that are vaccinated and naturally colonised by wild-type strains of *Salmonella* Enteritidis and *typhimurium.*

To generate the rough mutants, an experimental design was used based on an allelic exchange caused by a temperature-sensitive plasmid carrying two regions homologous to the adjacent 5 and 3' regions of the waaL gene. In order to carry out the allelic exchange by means of homologous recombination, two fragments of about 500-bp flanking waaL at regions 3' and 5' (fragments AB and CD) were first cloned into this plasmid. The constructed plasmid was introduced into strain 3934vac by electroporation (the strain of *Salmonella* carrying this plasmid is grown at 28 °C in the presence of chloramphenicol 20 µg/ml). Once the strain was transformed, several clones were selected for integrating the plasmid into the chromosome by means of culturing at 42 °C (non-permissive temperature for plasmid replication). After confirming integration of the vector into the chromosome by PCR, the cleavage or second recombination was performed. In this second step, the plasmid is lost with the desired deletion having been generated or the wild-type copy of the gene having been restored (the approximate ratio of these genetic events is 50-50 %). The second recombination is achieved by culturing at 30 °C in the absence of antibiotic and in the presence of sucrose, since the plasmid carries the lethal *sacB* gene and the presence of this sugar in the culture medium counterselects the clones still retaining the plasmid. Confirmation of those clones in which the allelic exchange occurred was performed by PCR and later sequencing. It should be pointed out that the mutants resulting from this method do not carry any resistances to antibiotics or traces of exogenous DNA.

To integrate an expression cassette into the chromosome that allows the fiber antigen to be produced in the strain of SE, an approach similar to that described earlier was used. In this case, the plasmid contains, besides the recombination flanking regions, an expression cassette that is integrated into the chromosome. This expression cassette was cloned into an integrative plasmid carrying regions AB and CD of about 500 bp of the Sb13 gene of the prophage ST64B. The cloning was designed so that the expression cassette of the Ava-I fiber is located between both fragments of the gene of the defective prophage. (The strain of *Salmonella* carrying this plasmid is grown at 28 °C in the presence of chloramphenicol 20 µg/ml). Once the plasmid was transformed by electroporation, several clones were subjected to several growth passages at 42 °C (non-permissive temperature of plasmid replication- plasmid integration occurs as a result of homologous regions AB and CD adjacent to waaL). After checking by PCR that the plasmid had been integrated into the chromosome, the cleavage or second recombination was carried out at 28 °C in the absence of antibiotic and in the presence of sucrose. As described earlier, this counterselection system allows identifying clones that have been lost in the plasmid. The last step again consists of selecting by PCR the clones into which the insertion of the expression cassette of fiber has occurred in the sb13 gene of prophage ST64B.

The resulting strain, the strain to which this invention relates, is a modified mutant strain of *Salmonella* Enteritidis carrying a deletion of the waaL gene, and therefore presents a rough phenotype and expresses fiber genes of Ava-I from its own chromosome.

For purposes of the present invention, *Salmonella* Enteritidis is an abbreviation of Salmonella enterica serovar Enteritidis.

Likewise, in the present invention PCR is understood to mean polymer chain reaction, and oligo is the primer used in PCR.

### DETAILED DESCRIPTION OF THE INVENTION

### PRODUCTION OF ATTENUATED SALMONELLA ENTERITIDIS (ASE)

### Design and construction of a system that allows introducing any xenoantigen of interest into the chromosome of Salmonella Enteritidis (SE).

A live vaccine or vector vaccine must be safe and effective, with a completely controlled genotype and phenotype which prevent the risk of reversion to virulence. Furthermore, the strain must maintain a balance between the degree of attenuation and immunogenicity, remaining in the organism of the host long enough to give rise to a protective immune response against homologous and/or heterologous antigens. In this case, the main feature of the generated strain of avian *Salmonella enteritidis* (ASE) is a drastic attenuation in poultry. Furthermore, the ASE strain is unable to form biofilms and has very low survival in the environment, preventing any risk associated with the period in which the vaccinated animals may excrete this strain. Unlike most commercial vaccines (such as 9R), its genotype and phenotype are completely controlled and do not possess antibiotic resistance genes. The harmlessness of live attenuated bacteria has been verified in other models, such as the 9R vaccine, with reports on same scientifically ruling out a potential reversion to the original virulent form (Okamoto., et al. 2010 Revista Brasilera de Ciencia Avícola).

Therefore, the present invention uses a strain of Salmonella enteritidis 3934 (deposited in the *Colección Española de Cultivos Tipo* (Spanish Type Culture Collection, CECT) under accession number CECT 7236), from which the twelve genes encoding enzymes diguanylate cyclases, the *rpoS* gene and the *waaL* gene have been deleted by genetic engineering techniques. This last mutation was carried out with a view to obtain a rough phenotype vaccine strain (Salmonella enteritidis 3934vac DwaaL) conferring protection in animals for breeding. Likewise, another aspect of the present invention comprises a rough phenotype vaccine strain carrying an expression cassette for fiber genes of Ava-I (Salmonella enteritidis 3934vac DwaaL -fiber) which confers immunity against inclusion body hepatitis in poultry.

### GENERATION OF ROUGH MUTANTS

With a view to reducing toxicity and maintaining immunogenicity of the strains based on the 3934vac genetic background, the *waaL* gene was deleted. The mutation of this gene translates into a rough phenotype due to the fact that the lipopolysaccharide is made up of only a core and lipid A, having lost the capacity to synthesise the O antigen. This mutation has the additional advantage of allowing the differentiation of animals that are vaccinated and naturally colonised by wild-type strains of *Salmonella* Enteritidis and *typhimurium.*

### Detailed Methodology

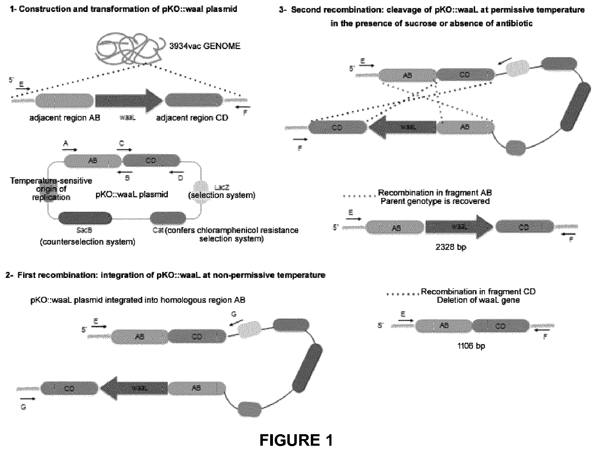

The methodology used consists of constructing a pKO:waaL integrative vector, integrating this pKO:waaL vector in a first recombination step and then cleaving the integrative vector with a second recombination step (Figure 1)

### 1. Construction of the pKO:waaL integrative vector

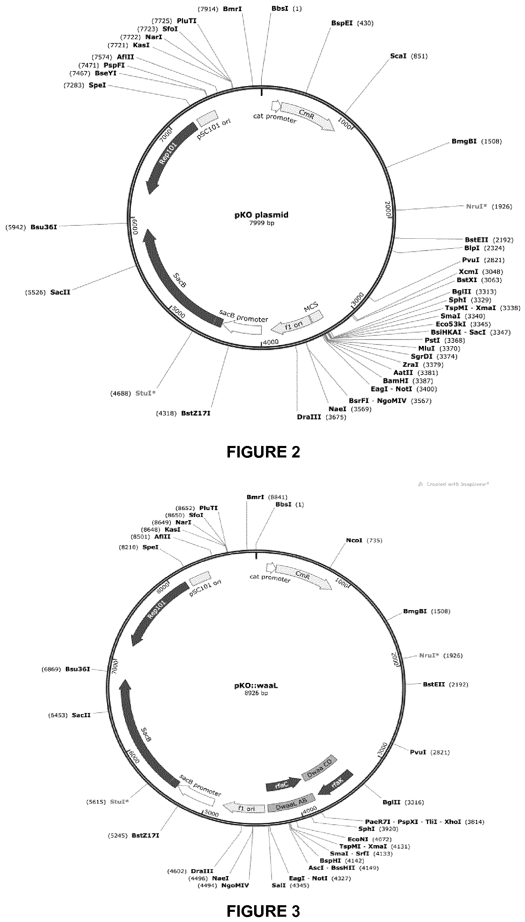

For constructing the pKO::waaL integrative plasmid, two fragments flanking the 520-bp waaL gene (oligonucleotides A and B) and 504-bp waaL gene (oligonucleotides C and D), respectively, are amplified by PCR. The PCR products are gel purified and cloned independently into standard cloning vectors, to then be digested into NotI XhoI (fragment AB) and XhoI BglII (fragment CD). Both digested fragments are gel purified and subcloned into the pKO plasmid the sequence of which is SEQ ID NO:1 digested into Notl BlgII, giving rise to the pKO::waaL vector. The pKO plasmid carries a chloramphenicol resistance cassette, a temperature-sensitive origin, a *lacZ* selection system and a *sacB* counterselection system (Figure 2)

The pKO::waaL construction, the sequence of which is SEQ ID NO:2, is performed in a strain of *Escherichia coli* and verified by PCR, miniprep and digestion.

The oligonucleotides used for constructing the pDEST::waaL vector are shown in Table 1. Sequence (5'-3'). The restriction sites are underlined.

**TABLE 1: Oligonucleotides used to perform deletion of the waaL gene in Salmonella enteritidis 3934**

| Name of the gene | Oligonucleotides |
|---|---|
| waaL | oligo A (SEQ ID NO: 3) |
| | oligo B (SEQ ID NO: 4) |
| | oligo C (SEQ ID NO: 5) |
| | oligo D (SEQ ID NO: 6) |

### 2. Integration of the pKO::waaL suicide vector (first recombination)

Once constructed and verified, the pKO::waaL plasmid (Figure 3) is extracted from the strain of *Escherichia coli* by means of miniprep and electroporated into strain *S.* Enteritidis 3934vac, where the integration thereof is forced by growth at 42 °C. This temperature is non-permissive for plasmid replication and plasmid integration occurs as a result of homologous regions AB and CD adjacent to waaL. Several chloramphenicol-resistant clones resulting from incubation at 44 °C were tested by PCR with oligo pairs E - F and E - G to verify integration. In the first case, the PCRs must be negative, and in the second case, those clones in which recombination has taken place in fragment AB, the resulting PCR will be of 1282 bp.

**TABLE 2: Oligonucleotides used for verification of the integration of the pKO::waaL plasmid**

| **Name of the oligo** | **Sequence** |
|---|---|
| oligo E | SEQ ID NO: 9 |
| oligo F | SEQ ID NO: 10 |
| oligo G (hybrid in the pKO plasmid) | SEQ ID NO: 11 |

### 3. Cleavage of the pKO::waaL integrative vector (second recombination)

With a view to the second recombination event occurring and the pKO plasmid being lost, several integrated clones are grown in liquid medium at 30 °C in the absence of antibiotic. After 24 hours, 5 serial dilutions are plated in solid medium supplemented with 5 % sucrose. In this medium, only those clones that have lost the plasmid through a second recombination grow which, depending on the fragment in which it has taken place, gives rise to the regeneration of the parent genotype or total deletion of the waaL gene. Both alleles can be differentiated by PCR with oligos E and F (sequence described earlier)

In parent strain 3934vac, PCR with oligos E and F gives rise to a fragment of 2328 bp, the sequence of the amplicon originating from the 3934vac genotype is SEQ ID NO:7, whereas in the clones in which deletion has occurred the fragment has a size of 1106 bp, the amplicon sequence of which is SEQ ID NO:8.

Strain 3934vacDwaal or 3934vacR can be grown at 37 °C in the absence of antibiotic since it carries no selection cassette.

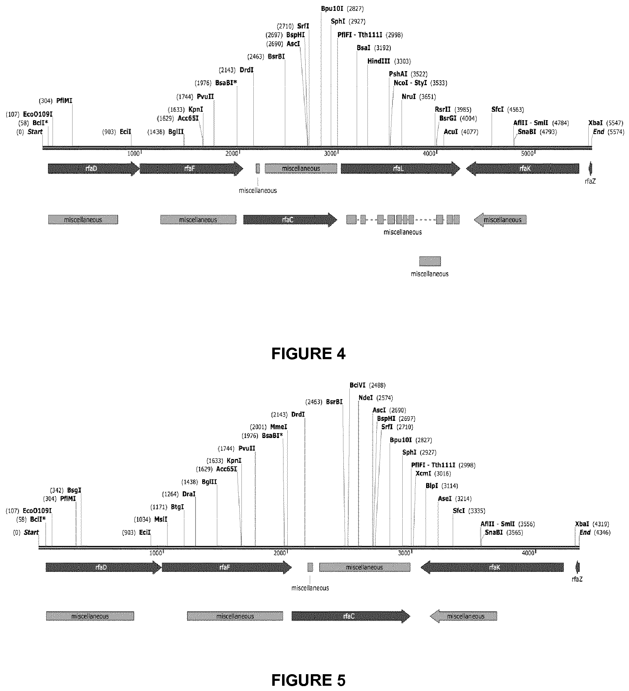

The Waa (rfa) region of Salmonella enteritidis 3934vac and the Waa (rfa) region of Salmonella enteritidis 3934vacR can be seen in the Figure 4 and Figure 5, respectively.

The oligonucleotides used in the verification of the mutants DwaaL are: SEQ ID NO:9 and SEQ ID NO:10

### CONSTRUCTION AND INSERTION OF CASSETTE WITH FIBER GENES OF AVA-I INTO STRAIN 3934VacR

### Construction and insertion of expression cassette with fiber genes of AvA-I into the chromosome of 3934VacR.

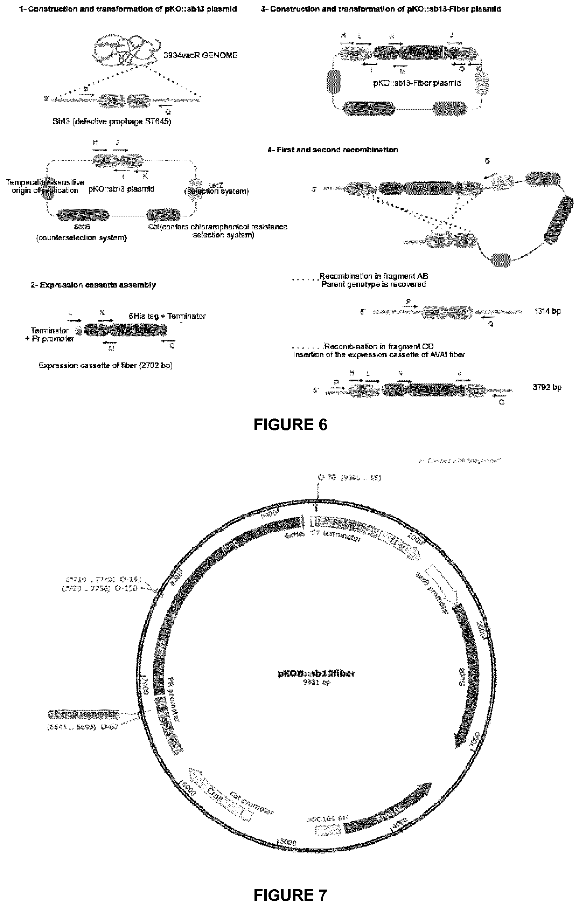

The insertion of the genes of interest into strain 3934vacR is performed at the chromosomal level. This strategy allows the expression of heterologous genes to be stable, eliminating the problem of the segregation of plasmids and the need to use antibiotics as selection markers. The steps are summarised in Figure 6.

### 1. Construction of the pKO:sb13-Fiber integrative vector

In this first phase, an integrative plasmid is constructed for integrating the expression cassette into the sb13 gene of defective prophage ST64B (all the strains of SE have it in their chromosome) (Figure 6). Following an approach similar to the one described earlier, regions AB (427 bp) and CD (597 bp) are amplified with oligo pairs H-I and J-K. The PCR products are gel purified and cloned independently into standard cloning vectors, to then be digested into SmaI sphI (fragment AB) and spHI SalI (fragment CD). Both digested fragments are gel purified and subcloned into the pKO suicide plasmid digested into SmaI SalI whose, giving rise to the pKO::sb13 vector (Figure 7) the sequence of which is SEQ ID NO: 12.

### Oligos used

Fragment AB:
   oligo H (SEQ ID NO:13)
   oligo I (SEQ ID NO:14)
Fragment CD:
   oligo J (SEQ ID NO:15)
   oligo K (SEQ ID NO:16)

Once the pKO::sb13 plasmid is constructed, the cassette that will give rise to expression of the fiber antigen is assembled. This step is carried out in several phases:(i) Amplification of <Terminator(43 bp)-Pr.promoter.RBS(114 bp)-clyA(915 bp)> from a synthetic plasmid with oligonucleotides L- M, (ii) amplification of the encoding region of the fiber antigen (1597 bp) using the pET28::fiber plasmid with the oligonucleotide pair N-O as a template, and (iii) overlapping PCR for fusing both fragments, with external oligos L-O.
Oligo L(SEQ ID NO:17)
Oligo M (SEQ ID NO: 18)
Oligo N (SEQ ID NO: 19)
Oligo O (SEQ ID NO: 20)

The expression cassette of fiber has sequence SEQ ID NO:21.

The resulting fragment (2,702 bp) is gel purified and cloned by the assembly of homologous ends into the pKO::sb13 vector previously digested into spHI, obtaining the pKO::sb13-Fiber vector the sequence of which is SEQ ID NO:22.

### 2. Integration of the pKO::sb13-fiber suicide vector (first recombination)

Once constructed and verified, the pKO::sb13-fiber plasmid is extracted from the strain of *Escherichia coli* by means of miniprep and electroporated into strain S. Enteritidis 3934vacR (or 3934vac DwaaL) where the integration thereof is forced by growth at 42 °C. This temperature is non-permissive for plasmid replication and plasmid integration occurs as a result of homologous regions AB and CD of sb13 gene. Several chloramphenicol-resistant clones resulting from incubation at 44 °C are tested by PCR with oligo pairs P-Q and P-M to verify integration. In the first case, the PCRs must be negative, and in the second case, those clones in which recombination has taken place in fragment AB, the resulting PCR will be of 1607 bp.

The oligos used were:
Oligo P (SEQ ID NO: 23)
Oligo Q (SEQ ID NO: 24)
Oligo M (SEQ ID NO: 25)

### -Cleavage of the pKO::ab13-fiber integrative vector (second recombination)

With a view to the second recombination event occurring and the pKO plasmid being lost, with the expression cassette of the fiber antigen being inserted into the chromosome, several integrated clones are grown in liquid medium at 30 °C in the absence of antibiotic. After 24 hours, 5 serial dilutions are plated in solid medium supplemented with 5 % sucrose. In this medium, only those clones that have lost the plasmid through a second recombination grow which, depending on the fragment in which it has taken place, gives rise to the regeneration of the parent genotype or the insertion into the sb13 gene of the defective prophage ST64B of the expression cassette responsible for the production of the fiber antigen. These last clones can be differentiated by PCR using oligos P and Q (sequence described earlier).

In parent strain 3934vacR, PCR with oligos P and Q gives rise to a fragment of 1314 bp, whereas in the clones in which strain 3934vacR::FIBER(Ava-I) has been produced, integration of the expression cassette of the fiber has a size of 3792 bp.

The sequence in the 3934vacR genetic background is SEQ ID NO:25.

The sb13 sequence in the 3934vacR::FIBER (Ava-I) genetic background is SEQ ID NO:26.

### Detection of the expression of fiber proteins by ASE

To confirm the heterologous expression of the fiber antigen, SDS-PAGE and Western blot techniques are used. Heterologous proteins are expressed by culturing the bacteria in LB medium, then proteins are extracted from the cytoplasm, periplasm and secreted. The samples obtained from the expression are separated by SDS-PAGE in 10% polyacrylamide gels. The proteins are transferred to nitrocellulose membranes and the proteins are detected indirectly using IgG-HRP (antibody bound to horseradish peroxidase) as a conjugate, followed by detection using DAB (3,3' Diaminobenzidine tetrahydrochloride) as a substrate.

Thus, the results obtained by Western blot satisfactorily validate and corroborate the expression of the fiber antigen in strain *Salmonella Enteritidis* 3934Vac under growth conditions both at 37 °C and at 28 °C. Even though none of the bands (A, B and C) coincides exactly with the estimated theoretical molecular weight of fusion ClyA-fiberAVAI-His (85.4 kDa), band A probably coincides with the complete size of the fusion, whereas bands B and C are protein degradation products. Where ClyA represents the sequence of the ClyA gene, fiberAVAI represents the sequence of the fiber gene of AVA-I, and His represents the sequence of 6 consecutive histidines.

### Phenotypic study of strains 3934 and rough vaccine derivatives.

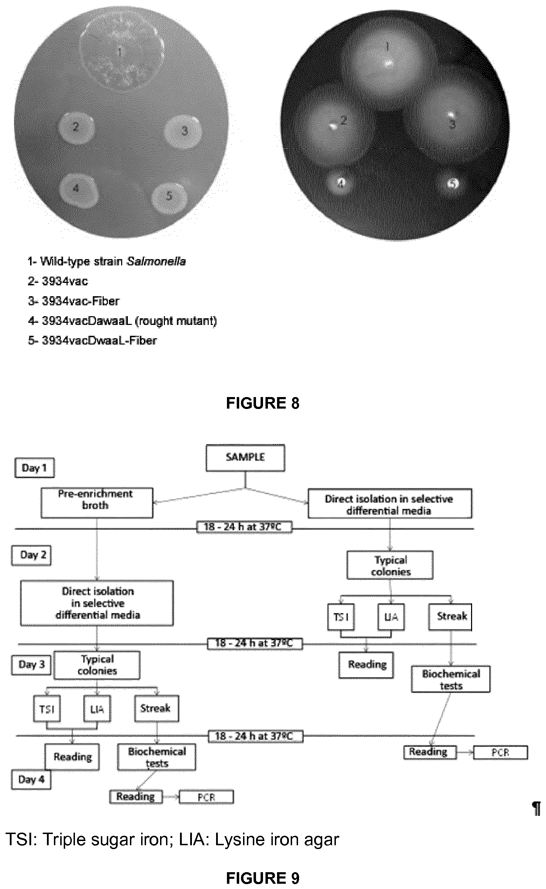

To study the phenotypes of the different strains, said strains were seeded in Congo red agar, which allowed simply differentiating the strain of salmonella carrying the fiber gene. Unlike wild-type strains, which normally produce cellulose, fimbriae and other appendages, giving rise to the RDAR-*red*, *dry and rough-* genotype, the mutants generated in the present invention have a phenotype which is known as *saw (smooth and white).* This phenotype is particularly notable when the strains produce a truncated lipopolysaccharide due to the mutation in the waaL gene. The differences are also significant in semisolid *swimming* agar, although this phenotype can sustain variations depending on the exact composition and humidity of the culture plates (Figure 8).

### EVALUATION OF THE RECOMBINANT VACCINE

### Immunisation and challenge tests

### Preparation of the inoculum of the vaccine.

The inoculum is prepared from strain RAvA-SE which suitably expresses the fiber gene of AvA-I. The bacterium is seeded in XLD medium and incubated for 24 hours at 37 °C. Typical colonies are transferred to LB medium, the culture is incubated for 18 hrs at 37 °C under stirring at 250 rpm (mechanical stirrer). The bacterial cells are collected by centrifugation at 5000 g for 10 min and serially diluted in PBS according to the desired concentration.

### Preparation of the inoculum of AvA-I for challenge tests.

The AvA-I isolate used in the test was obtained from an IBH (inclusion body hepatitis) outbreak on a farm in Chincha, Ica, Peru, characterised as AvA-I serotype 4 by RFLP-PCR. The isolate is inoculated in 10 day-old SPF eggs through the chorioallantoic membrane (CAM), and they are incubated for 9 days, at the end of which time the livers are collected to be homogenised and the viral load is determined by PCR. The mean infectious dose in chicken embryo (IDCE50/ml) of the inoculum is calculated using the Reed and Muench method. The homogenised material is stored at -20°C until it is used in the challenge.

### Protection test

At this point, testing with animals will allow evaluating the immunogenicity of the RAvA-SE candidate vaccine. A similar timeline such as the one shown below is established for that purpose. The number of work groups is established according to the dosage and route of administration. If more than one candidate vaccine is generated, the same scheme will also be followed. Each treatment group consists of 30 animals, and this number is justified according to the timeline which involves sacrificing the animals throughout the experiment to evaluate colonisation of the bacterium in their internal organs. Animals are to be understood as poultry for breeding.

A standardised model vaccination schedule was followed according to the following timeline

**TABLE 3: Timeline for vaccination schedules**

| **Timeline** | **Type of samples** | **Laboratory** | **(ELISA, PCR, Culture)** | **Day** | | | | |
|---|---|---|---|---|---|---|---|---|
| | | | | **0** | **7** | **14** | **21** | **28** |
| **Date** | | | | | | | | |
| **Age of the animal** | | | | 1 day | 8 days | 15 days | 22 days | 29 days |
| **Vaccine 1^{st} dose** | | | | X | | | | |
| **Challenge** | | | | | | | X | |
| **Blood** | serum | MSL | ELISA | X | X | X | X | X |
| **Organs** | liver, spleen, feces, swabbing | MBGL/IMS | PCR/Microbiological culture | X | X | X | X | X |
| **Others** | | | | | | | | |
| MSL: Microbiology and Serology Laboratory | | | | | | | | |
| MBGL: Molecular Biology and Genomic Laboratory | | | | | | | | |

One day-old SPF Hyline hens are used in this point, which are vaccinated according to the aforementioned schedule. After vaccination, the animals are monitored to observe clinical signs. Each experimental group is kept in separate cages. After day 21, the animals are challenged intramuscularly with 10¹⁰ copies/ml of AvA-I serotype IV, which is equivalent to 10⁵ IDCE₅₀ (mean infectious dose in chicken embryo). One of the groups is challenged both with AvA-I (intramuscular route), and with a wild-type strain of *Salmonella* enteritidis (oral route, 10⁷ copies/ml). Samples are taken at regular intervals, according to the established timeline, which will include feces, sewer swabs, liver and spleen for the detection and quantification of RAvA-SE, wild-type SE and AvA-I. Blood samples are also considered according to the timeline for measuring protective antibodies. At 7 days post-challenge, all the animals are euthanised.

### Determination of excretion and colonisation of attenuated Salmonella enteritidis in chickens

Testing with animals at this point allows determining that the strain of attenuated Salmonella enteritidis (ASE) affords the characteristics according to which it has been designed: avirulent, reduced colonisation capacity, persistence and excretion, compared with a wild-type field strain.

The work timeline is designed specifying: treatment groups, route of administration, concentration of the bacterium to be used, type of sample to be evaluated and sampling days. Each treatment consists of 15 animals, a number that contemplates sacrificing the animals on the days indicated in the timeline to evaluate colonisation of the bacterium in their internal organs. One day-old animals separated by group according to treatment are inoculated with strain ASE using a 1 ml sterile syringe. The animals are monitored daily to observe clinical signs and are administered food and water *ad libitum.* Relative humidity conditions and suitable light-dark cycles are maintained. At the end of the experiment, the animals are euthanised by electric shock.

According to the design of the experiment, samples are sent to the laboratory for the respective microbiological, serological and molecular analyses.

### Microbiological assays for the detection and quantification of Salmonella.

These assays allow determining excretion and tissue colonisation of the bacterium ASE throughout the experiment, according to the established sample shipping timeline.

Feces, rectal swab, spleen and liver samples from poultry from the different evaluated groups are analysed throughout the experiment according to the established sample shipping timeline. The method for isolation is carried out as indicated in the flow chart (Figure 9).

The bacterial load in the samples was determined.

### LIST OF FIGURES

Figure 1: Allelic exchange method for the construction of 3934vacR.
Figure 2: pKO integrative plasmid
Figure 3: pKO::waaL integrative plasmid
Figure 4: Waa (rfa) region of Salmonella Enteritidis 3934vac
Figure 5: Waa (rfa) region of Salmonella Enteritidis 3934vacR
Figure 6: pKOB::sb13 plasmid
Figure 7: pKO B::sb13fiber plasmid
Figure 8: Phenotypic differentiation of strains of Salmonella in Congo red agar plates (left) and in semisolid agar (right).
Figure 9: Flow chart for the microbiological detection and quantification of Salmonella. Furthermore, TSI: Triple sugar iron agar; LIA: Lysine iron agar.
Figure 10: Western blot results for the protein of interest clyA-fiberFAdV-His.
Figure 11: SDS-PAGE results for the protein of interest clyA-fiberFAdV-His, with positive controls A and B.
Figure 12: Coomassie staining of the SDS-PAGE and Western blot of the protein extracts obtained after growth in LB medium at 37 °C or 28 °C. Furthermore, MW: molecular weight marker, wt: Salmonella Enteritidis 3934, Δ: Salmonella Enteritidis 3934Vac; Δ-fiber: Salmonella Enteritidis 3934Vac with fusion ClyA-fiberFAdV-His in the chromosome, Δ-pfiber: Salmonella Enteritidis 3934Vac expressing fusion ClyAfiberFAdV-His from the plasmid. The specific bands obtained in the Western blot are indicated (A, B and C).
Figure 13: Electrophoretic analysis of the PCR products reveals the presence of clones carrying the *waaL* deletion. For the selection of clones carrying the deletion, the bacterial chromosome was amplified using oligos external to the construction. Furthermore, M = molecular weight marker, wt = wild-type strain, c = colony. Expected sizes: Wt = 2328 bp, Δ*waaL*=1112 bp

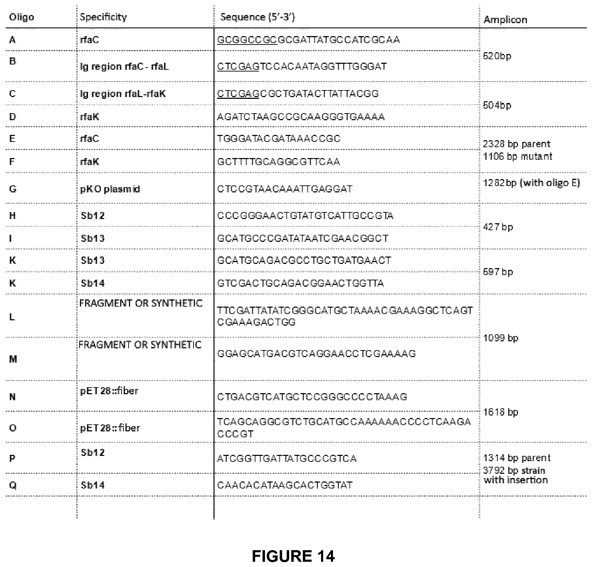

Figure 14: Table summarising the oligos used

### PREFERRED EMBODIMENT OF THE INVENTION: EXAMPLES

The following examples provided herein serve to illustrate the nature of the present invention. These examples are included only for illustrative purposes and must not be interpreted as limitations to the invention herein claimed.

### Example 1: Expression of the fiber antigen in strain Salmonella Enteritidis 3934 Vac by means of Western blot.

### Protein extract

One colony was seeded in 5 ml of LB culture medium for ClyA fiber q which has the insertion into the chromosome. It is incubated at 37 °C at 200 rpm overnight.

It is centrifuged at 11 0000 rpm for 5 minutes, and lysis 1 buffer (10 Mm TrisHCL, 5 mM EDTA, 50 mM NaCl) + 40 ul of protease inhibitor (3 gr/ml) + 50 ul of lysozyme (10 mg/ml), plus SDS loading buffer (50 ul of simple buffer 2x + 50 ul of 8M urea + 5 ul of β-mercaptoethanol in 50 ul of sample) are added per sample and it was all homogenised and heated at 100 °C for 5 minutes and then on ice for 5 minutes, then loading 20 ul; it was taken to be charged. 0.1 amperes for each gel. At 1:30.

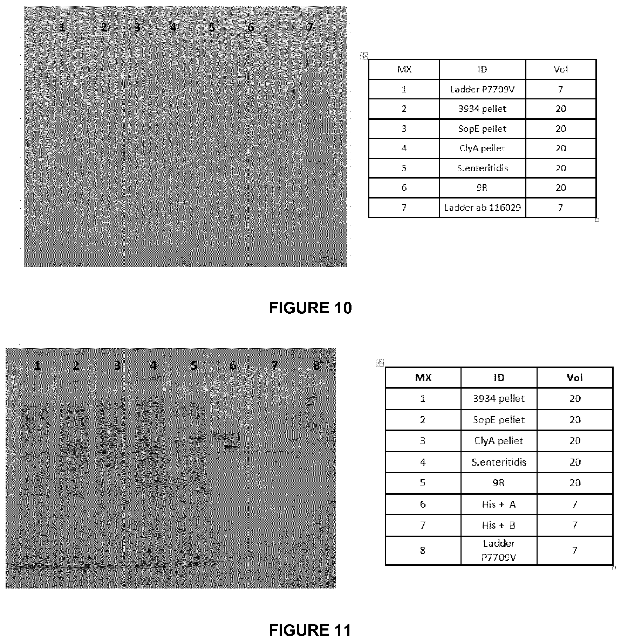

The band of the protein of interest clyA-fiberFAdV-His was observed in the Western blot results. Markers were used; the one for the left being ladder P7709V (175 KDa), and the one for the right being ab 116029 (245 KDa). (Figure 10)

Furthermore, when two positive controls of His+ are used, but the initial A and B is by weight, A is about 60 kDa and B is about 30kDa. These results allow concluding that fusion clyA-fiberFAdV-His is expressed with a molecular weight of about 85.4 kDa. (Figure 11)

Western blot was performed for wild-type strain: Salmonella Enteritidis 3934, Δ: Salmonella Enteritidis 3934Vac; Δ-fiber: Salmonella Enteritidis 3934Vac with fusion ClyA-fiberFAdV-His in the chromosome, Δ-pfiber: Salmonella Enteritidis 3934Vac expressing fusion ClyAfiberFAdV-His from the plasmid.

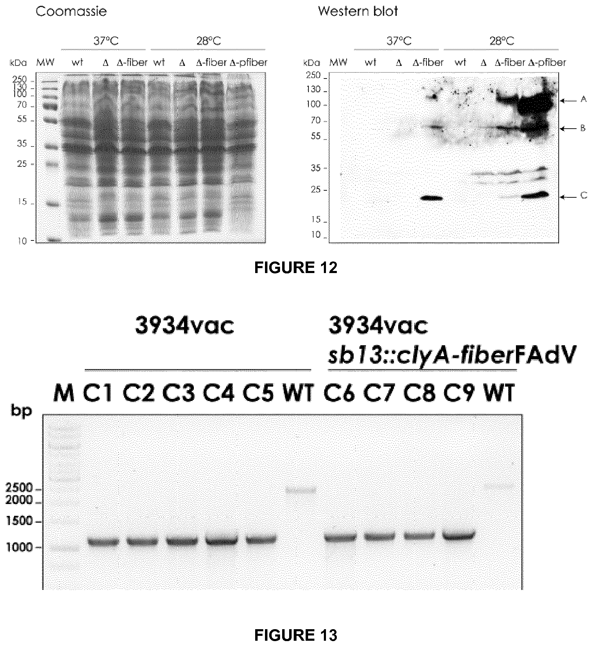

Electrophoretic analysis of the PCR products reveals the presence of clones carrying the *waaL* deletion. (Figure 13)

For purposes of the present invention, the use of strain Salmonella enteritidis 3934vac in experimental models showed that it would work in a murine and even an avian model.

In the present testing it could be seen that when poultry is inoculated it does not work well because they develop infection in any case; in order to achieve immunity, a deletion in the waaL gene of Salmonella enteritidis 3934vac was performed, thereby achieving immunity. Likewise, challenge tests with strain 3934vac sb13::clyA-fiberFAdV ΔwaaL demonstrated that it confers immunity against Salmonella and avian Adenovirus type-I, preventing the occurrence of inclusion body hepatitis.

### Deposit of microorganisms

Strains SG-9R sb:ClyA-FIBER 6His, rough 3934 vac-mutant and rough 3934 vac-Fiber mutant have been deposited in the *Colección Española de Cultivos Tipo* (Spanish Type Culture Collection, Paterna, Valencia, Spain), following the rules of the Budapest Treaty for the deposit of microorganisms for patenting purposes on the following dates, and they were assigned the following deposit number.

| Material | Date of Deposit | Accession Number |
|---|---|---|
| SG-9Rsb:ClyA-FIBER 6His | 5 April 2017 | CET 9331 |
| Rough 3934 vac-mutant | 5 April 2017 | CET 9332 |
| Rough 3934 vac-Fiber mutant | 5 April 2017 | CET 9333 |

The present invention is not limited to the scope of the microorganisms deposited in the patent given that they represent a mere illustration of one aspect of the invention. Any microorganism or plasmid that is functionally equivalent to those described in the invention are included within the invention.

## Claims

1. A mutant strain of Salmonella enteritidis 3934vac, **characterised in that** it comprises a deletion of the waaL gene.

2. A method for generating a mutant strain of Salmonella enteritidis 3934vac, **characterised in that** it comprises:
• Constructing an integrative vector, wherein for the construction of the integrative vector two fragments flanking the 520-bp waaL gene (oligonucleotides A and B) and 504-bp waaL gene (oligonucleotides C and D), respectively, are amplified by PCR, wherein oligonucleotides A, B, C and D are sequences SEQ ID NO: 3, SEQ ID NO: 4, SEQ ID NO: 5, SEQ ID NO: 6;
• Purifying and cloning the PCR products separately into pKOB vectors for amplification thereof to obtain the resulting pKOB::DwaaLR1 vector for amplification thereof, then enzymatically digesting, purifying and binding in the pDESTINATION vector;
• Transforming the resulting pDEST::waaL vector into Escherichia coli and verifying by PCR, miniprep and digestion, wherein once constructed, the pDEST::waaL plasmid is electroporated into strains S. Enteritidis 3934vac and 3934vac sb13::clyA-fiberFAdV, wherein the integration thereof is forced by growth at 42 °C and subsequent cleavage at 28 °C;
• Verifying by PCR with oligos external to the construction (waaL E and F) the deletion of the waaL gene by means of double homologous recombination, wherein oligos E and F are SEQ ID NO: 9 and SEQ ID NO: 10.

3. A recombinant avian vaccine of Salmonella enteritidis, **characterised in that** it comprises a modified mutant strain of Salmonella enteritidis 3934 vac comprises a deletion of the waaL gene.

4. The mutant strain of Salmonella enteritidis 3934vac **characterised in that** it further comprises the complete sequence of the fiber gene of AVA-I and a deletion of the waaL gene.

5. The method for generating a mutant strain of Salmonella enteritidis 3934vac according to claim 1, **characterised in that** it comprises:
• Having a strain of Salmonella enteritidis 3934 vac;
• Inserting a selection cassette by means of a first homologous recombination step, wherein the cassette contains, in addition to the antibiotic resistance gene, at its flanking regions, sequences complementary to prophage ST64B; wherein in the first homologous recombination step the bacterium is electroporated in the presence of linear DNA carrying the selection cassette;
• Selecting the transforming colonies, after the first homologous recombination step, in LB medium containing the antibiotic;
• Confirming the presence of the cassette in the chromosome of Salmonella enteritidis 3934 by PCR with the oligos of sequences SEQ ID NO: 23, SEQ ID NO:24 and SEQ ID NO: 25;
• Inserting an expression cassette of ClyA-fiberFAdV-His by means of a second homologous recombination step, wherein the expression cassette replaces the selection cassette, wherein the expression cassette comprises the sequence SEQ ID NO:21;
• Selecting the strains of Salmonella enteritidis 3934vac sb13::clyA-fiberFAdV by verifying the product of the expression of the sequence SEQ ID NO:21 by Western blot and SDS-PAGE.

6. A method for generating a mutant strain of Salmonella enteritidis 3934vac, **characterised in that** it comprises:
• Constructing an integrative vector, wherein for the construction of the integrative vector two fragments flanking the 520-bp waaL gene (oligonucleotides A and B of sequences SEQ ID NO: 3 and SEQ ID NO: 4, respectively) and 504-bp waaL gene (oligonucleotides C and D of sequences SEQ ID NO: 5, SEQ ID NO: 6, respectively) are amplified by PCR;
• Purifying and cloning the PCR products separately into pKOB vectors for amplification thereof to obtain the resulting pKOB::DwaaLR1 vector for amplification thereof, then enzymatically digesting, purifying and binding in the pDESTINATION vector;
• Transforming the resulting pDEST::waaL vector into Escherichia coli and verifying by PCR, miniprep and digestion, wherein once constructed, the pDEST::waaL plasmid is electroporated into strains S. Enteritidis 3934vac and 3934vac sb13::clyA-fiberFAdV, wherein the integration thereof is forced by growth at 42 °C and subsequent cleavage at 28 °C;
• Verifying by PCR with oligos external to the construction SEQ ID NO: 9 and SEQ ID NO: 10, the deletion of the waaL gene by means of double homologous recombination.

7. The recombinant avian vaccine of Salmonella enteritidis **characterised in that** it comprises a modified mutant strain of Salmonella enteritidis 3934 vac with a sequence of the fiber gene of AVA-I and a deletion of the waaL gene.

8. A mutant strain of Salmonella enteritidis 3934 vac **characterised in that** it comprises an expression cassette SEQ ID NO:21 and the deletion of waaL, which is verified by the presence of the sequence SEQ ID NO:8.

9. A mutant strain of Salmonella enteritidis 3934 vac **characterised in that** it comprises a sequence SEQ ID NO:26 y SEQ ID NO:8.
